# FASCICULE DE BREVET EUROPEEN

(11) **EP 1 112 325 B1**
(45) Date de publication et mention de la délivrance du brevet: **07.05.2003**
(21) Numéro de dépôt: 00940464.1
(22) Date de dépôt: 08.06.2000
(51) Int. Cl.: C08L 71/02, C08L 5/00, A61K 7/02, A61K 7/027, C08L 101/00

(54) **GEL AQUEUX SOLIDE COMPRENANT UN GELIFIANT HYDROPHILE ET UN POLYETHYLENE GLYCOL PARTICULIER, COMPOSITION COMPRENANT CE GEL ET UTILISATIONS**
WÄSSRIGES, FESTES GEL ENTHALTEND EIN HYDROPHILES GELIERUNGSMITTEL UND EIN BESONDERES POLYETHYLENEGLYKOL, ZUSAMMENSETZUNG ENTHALTEND DIESES GEL UND VERWENDUNGEN
AQUEOUS SOLID GEL COMPRISING A HYDROPHILIC GELLING AGENT AND A PARTICULAR POLYETHYLENE GLYCOL, COMPOSITION COMPRISING SAME AND USES

(30) Priorité: 18.06.1999 FR 9907766
(43) Date de publication de la demande: 04.07.2001
(73) Titulaire: L'OREAL, 75008 Paris (FR)
(72) Inventeur: BARA, Isabelle, F-75013 Paris (FR)
(74) Mandataire: Doressamy, Clarisse
(86) Numéro de dépôt international: FR0001577
(87) Numéro de publication internationale: WO00078868

(56) Documents cités:
- EP-A- 0 803 245
- "GELATIN-FREE SYSTEM FOR SOFT/HARD CAPSULES CONTAINING GELLAN GUM" RESEARCH DISCLOSURE,GB,INDUSTRIAL OPPORTUNITIES LTD. HAVANT, no. 332, 1 décembre 1991 (1991-12-01), page 908 XP000274618 ISSN: 0374-4353

## Description

La présente invention concerne un gel aqueux solide, une composition solide à phase continue aqueuse comprenant un tel gel et leur utilisation dans le domaine cosmétique, notamment pour le maquillage de la peau et/ou des muqueuses et/ou des fibres kératiniques.

On connaît dans l'industrie cosmétique des produits se présentant sous forme solide. Comme produits de ce type, on peut citer par exemple dans le domaine du maquillage, les bâtons ou "sticks" de rouge à lèvres, de fond de teint ou d'ombre à paupières ; dans le domaine du soin de la peau ou des lèvres, les crayons réparateurs des lèvres, les bâtons ou "sticks" dépigmentants, démaquillants ou hydratants ; dans le domaine de l'hygiène, les sticks déodorants, les sticks ou les pains moussants pour le rasage ou pour le lavage de la peau.

Il est en effet particulièrement intéressant de disposer de produits sous forme de sticks dans la mesure où de tels produits sont très pratiques à utiliser, ils sont facilement transportables, le produit ne risque pas de couler.

Par ailleurs, les produits de maquillage sont assez généralement formulés sur la base d'une part d'une phase grasse pour des raisons de confort et de douceur et d'autre part d'une phase pulvérulente qui apporte la couleur souhaitée. Cette phase pulvérulente peut comprendre des pigments et/ou des charges et/ou des nacres. La phase grasse comprend généralement des cires et/ou des huiles et/ou des composés pâteux.

Or, les sticks formulés à base de cires présentent certains inconvénients : ils ont un caractère gras qui n'est pas apprécié par les utilisateurs et ils manquent de fraîcheur à l'application. En outre, il est difficile d'y introduire des actifs hydrophiles.

On cherche donc de plus en plus à faire des sticks de maquillage comprenant une phase aqueuse dans la plus grande concentration possible. Toutefois, les sticks comprenant une phase aqueuse importante sont parfois sujets à des problèmes de stabilité et de manque de cohésion. En particulier, ces gels, qui sont réalisés à partir de l'association d'un gélifiant hydrophile et d'eau ont l'inconvénient d'être fragiles et peuvent se casser facilement lors de l'utilisation.

Un moyen d'améliorer la solidité des gels est d'augmenter la concentration en gélifiant hydrophile mais les gels deviennent alors difficilement délitables, c'est-à-dire que le prélèvement de matière lors de la prise de produit est insuffisante.

Or, il est primordial, pour un produit de maquillage en particulier, que le prélèvement du produit se fasse de façon idéale, c'est-à-dire permette, de façon simple, à l'aide du doigt ou d'une éponge ou même directement sur la peau du corps par exemple, de prendre non seulement la quantité de produit adéquate (pas trop pour ne pas perdre de produit inutilement mais suffisamment pour assurer un effet maquillage) mais également de conserver l'intégrité du produit au moment de son prélèvement : il ne faut pas casser le produit par un phénomène de cisaillement mais bien prélever l'ensemble du produit avec les pigments et/ou les nacres, et/ou éventuellement les charges, qui assurent la fonction du maquillage. Ce n'est qu'à cette condition que l'application du produit pourra se faire de façon homogène et que le maquillage obtenu sera uniforme.

Des produits délitables existent également mais ils sont alors trop mous et finissent par se cisailler lors d'applications répétées ou encore présentent des phénomènes de synérèse au cours du temps, c'est-à-dire que la partie liquide finit par s'exsuder et le produit présente deux phases : une phase solide indélitable et une phase liquide. Le produit ne peut plus assurer sa fonction, à savoir, maquiller, puisqu'il est impossible de prendre les pigments sur le doigt ou sur une éponge.

Aussi, il subsiste le besoin d'un gel aqueux solide pouvant être utilisé par application directe sur la peau ou à l'aide d'une éponge, délitant bien tout en restant suffisamment solide et non cassant au cours de l'utilisation.

La demanderesse a découvert de façon inattendue qu'en associant à un gélifiant hydrophile un polyéthylène glycol particulier, à savoir un polyéthylène glycol dont le nombre de moles d'oxyéthylène va de 12 à 180, il était possible de réaliser des gels aqueux solides homogènes et stables, présentant une excellente cohésion et pouvant se déliter facilement au doigt ou à l'éponge ou encore directement sur la peau du visage ou du corps.

En effet, la Demanderesse a constaté que l'association d'un polyéthylène glycol dont le nombre de moles d'oxyéthylène est inférieur à 12 à un gélifiant hydrophile diminuait fortement la dureté rendant par là même le délitage pratiquement impossible : le gel devient trop mou et le produit n'est plus utilisable en stick ou en coupelle. De même et de façon inexpliquée, l'association d'un polyéthylène glycol dont le nombre de moles d'oxyéthylène est supérieur à 180 à un gélifiant hydrophile entraîne également le ramollissement du gel qui de plus devient collant et n'est donc pas adapté à une utilisation cosmétique. Seule l'association revendiquée ci-après, avec un polyéthylène glycol particulier, permet d'obtenir une composition solide présentant des propriétés idéales à la fois de cohésion et de délitage.

La présente invention porte donc sur un gel aqueux solide comprenant i) au moins un gélifiant hydrophile et ii) au moins un polyéthylène glycol dont le nombre de moles d'oxyéthylène va de 12 à 180.

Les gels de l'invention présentent des qualités d'application et de délitage excellentes. En particulier, grâce à l'association selon l'invention, on obtient, à dureté équivalente, un niveau de délitage supérieur à celui des sticks connus. La prise du produit est facile, elle peut s'effectuer directement sur le corps ou avec le doigt ou encore à l'éponge, en prélevant une quantité suffisante de produit, facile à appliquer ensuite sur la peau de façon homogène, sans nécessiter de mouillage préalable. Le maquillage obtenu est uniforme et homogène.

Ces gels présentent par ailleurs une excellente cohésion. Ces gels sont stables dans le temps et à la température. Ainsi, après avoir été conservés deux mois à température ambiante ou à 45°C, ils ne présentent aucun phénomène de synérèse (exsudation) ou encore de déphasage : leur aspect et leur dureté n'ont pas varié.

Les gels selon l'invention n'exsudent pas, même à de faibles taux de gélifiant, et ils ne nécessitent pas obligatoirement l'intervention d'une technique de préparation particulière. Ils procurent à l'application une sensation de grande fraîcheur tout en conservant de bonnes propriétés cosmétiques, en particulier de douceur.

La présente invention a également pour objet une composition solide à phase continue aqueuse comprenant un gel tel que défini ci-dessus.

La présente invention a également pour objet un produit de maquillage de la peau ou des fibres kératiniques comprenant un gel et/ou une composition tels que définis ci-dessus.

La présente invention a encore pour objet un procédé de maquillage de la peau et/ou des muqueuses et/ou des fibres kératiniques, consistant à appliquer sur ces dernières, un gel aqueux et/ou une composition solides et/ou un produit de maquillage tels que définis ci-dessus.

Au sens de la présente invention, on entend par gel ou composition solide, un gel ou composition présentant une dureté définie par une force maximum avant rupture allant de 5 à 50 grammes, à température ambiante (20-25°C), après pénétration par un mobile en inox de 2 mm de diamètre dans la matrice du gel ou composition à une épaisseur de 1 mm à une vitesse de 1 mm/s et retrait dudit mobile de la matrice du gel ou composition à une vitesse de 2 mm/s ; la force maximum avant rupture étant mesurée avec un analyseur de texture du type "TAXT2" commercialisé par la Société RHEO.

De préférence encore, la force maximum avant rupture va de 7 à 40 g.

Le gel selon l'invention comprend un gélifiant hydrophile. Par gélifiant, on entend un composé qui, en présence d'un solvant, crée des liaisons intermacromoléculaires plus ou moins fortes induisant ainsi un réseau tridimensionnel qui fige ledit solvant.

Ce gélifiant hydrophile peut être choisi parmi les polysaccharides, les dérivés de protéines, les gels de synthèse ou d'hémisynthèse de type polyester, en particulier sulfonique, les polyacrylates ou polyméthacrylates et leurs dérivés.

Parmi les polysaccharides, on peut citer :
- les extraits d'algue tels que l'agar-agar, les carraghénanes (iota, kappa, lambda), les alginates, en particulier de Na ou Ca ;
- les exsudats de micro-organismes tels que la gomme de xanthane et ses dérivés comme le produit vendu sous la dénomination commerciale "Rhéosan" par la Société Rhodia Chimie, le gellane,
- les extraits de fruits tels que les pectines ;
- les agents gélifiants d'origine animale comme les dérivés de protéine, en particulier la gélatine, de boeuf ou de poisson, les caséïnates ;
- les polysaccharides possédant une chaîne latérale et 6 sucres neutres tels que décrits dans le document FR-A-2759377,
- et leurs mélanges.

Parmi les gels de synthèse ou d'hémisynthèse, on peut citer les copolyesters décrits dans la demande FR-A-2 760 643.

De préférence, le gélifiant hydrophile est choisi parmi les polysaccharides, et de préférence encore, le gélifiant hydrophile est le gellane.

Comme produits convenant particulièrement bien à l'invention, on peut citer la gomme de gellane vendue sous la dénomination commerciale « Kelcogel F » par la société NUTRASWEET-KELCO ou encore le iota carraghenane vendu sous les dénominations commerciales « Seaspen PF 357 » ou « Viscarin SD 389 » par la société FMC.

Le gélifiant hydrophile est présent dans le gel selon l'invention à une concentration permettant d'obtenir, en association avec polyéthylène glycol particulier, la dureté convenable pour un délitage idéal. Le gélifiant hydrophile est de préférence présent dans le gel selon l'invention à une concentration pouvant aller de 0,1 à 30%, de préférence encore de 0,2 à 10%, en poids, par rapport au poids total du gel.

Le gel selon l'invention comprend également un polyéthylène glycol dont le nombre de moles d'oxyéthylène va de 12 à 180. Les polyéthylène glycols sont des composés connus de formule suivante : H(OCH₂CH₂)ₙOH dans laquelle n représente le nombre de moles d'oxyéthylène. Des composés utilisables dans la présente invention sont par exemple les produits répertoriés dans le CTFA sous les noms "PEG-12", "PEG-32", "PEG-75, "PEG-180". On peut citer le polyéthylène glycol à 12 OE vendu sous le nom commercial « Polyéthylène 600 » par la société Lambert Rivière, le polyéthylène glycol à 180 OE vendu sous le nom commercial « Polyéthylène 6000 » par la société Kao Soap.

De préférence, le polyéthylène glycol utilisé dans la présente invention présente un nombre de moles d'oxyéthylène de 12.

Le polyéthylène glycol selon l'invention est présent dans le gel selon l'invention à une concentration permettant d'obtenir, en association avec le gélifiant hydrophile, la dureté et la consistance convenables pour un délitage idéal. De préférence le polyéthylène glycol selon l'invention est présent dans le gel selon l'invention à une concentration pouvant aller de 1 à 20% en poids, de préférence encore de 2 à 10% en poids, par rapport au poids total du gel.

Les gels de l'invention contiennent en outre un milieu cosmétiquement ou physiologiquement acceptable, c'est-à-dire un milieu compatible avec toutes les matières kératiniques telles que la peau, les ongles, les cheveux, les cils et sourcils, les muqueuses et les semi-muqueuses, et toute autre zone cutanée du corps et du visage.

Les gels selon l'invention peuvent également comprendre une eau florale telle que l'eau de bleuet et/ou une eau minérale telle que l'eau de VITTEL, l'eau de LUCAS ou l'eau de LA ROCHE POSAY et/ou une eau thermale.

Les gels selon l'invention peuvent également comprendre des colorants hydrosolubles choisis parmi les colorants usuels du domaine considéré tels que le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle.

De préférence, les gels selon l'invention comprennent jusqu'à 98,9% en poids, de préférence de 20 à 95% en poids, par rapport au poids total du gel, d'eau.

Les gels selon l'invention peuvent également comprendre des solvants autres que l'eau comme par exemple les alcools primaires tels que l'éthanol et l'isopropanol, les glycols tels que le propylène glycol, le butylène glycol, le dipropylène glycol, le diéthylène glycol, les éthers de glycol tel que les alkyl(C₁-C₄)éther de mono, di- ou tripropylène glycol, mono, di- ou triéthylène glycol, et leurs mélanges.

Il est possible de modifier la rigidité des gels selon l'invention en y ajoutant un ou plusieurs sels qui vont augmenter cette rigidité. Ces sels peuvent être choisis parmi les sels des métaux mono-, di- ou trivalents, et plus particulièrement les sels de métal alcalin et alcalino-terreux et en particulier les sels de sodium, de calcium ou de magnésium. Les ions constituant ces sels peuvent être choisis par exemple parmi les carbonates, les bicarbonates, les sulfates, les glycérophosphates, les borates, les chlorures, les nitrates, les acétates, les hydroxydes, les persulfates ainsi que les sels d'α-hydroxyacides (citrates, tartrates, lactates, malates) ou d'acides de fruits, ou encore les sels d'acides aminés (aspartate, arginate, glycocholate, fumarate). La quantité de sel peut aller de 0,01 à 2 % et de préférence de 0,1 à 1 % du poids total du gel.

De préférence, le sel est choisi parmi le nitrate de calcium, de magnésium ou de strontium, le borate de calcium ou de magnésium, le chlorure de calcium, de sodium, de magnésium, de strontium, de néodyme ou de manganèse, le sulfate de magnésium ou de calcium, l'acétate de calcium ou de magnésium, et leurs mélanges. De préférence encore, le sel est le chlorure de magnésium.

Le gel selon l'invention peut également comprendre une phase pulvérulente qui peut comprendre un pigment et/ou une nacre et/ou une charge.

Par pigments, il faut comprendre des particules blanches ou colorées, minérales ou organiques, insolubles dans le milieu, destinées à colorer et/ou opacifier la composition.

Les pigments peuvent être présents à raison de 0-40 % en poids, par rapport au poids total du gel, de préférence à raison de 0,1 à 30% et de préférence encore à raison de 1-20 %. Ils peuvent être blancs ou colorés, minéraux et/ou organiques, de taille usuelle ou nanométrique. Par taille nanométrique, on entend des pigments dont la taille moyenne des particules va de 5 à 100 nm.

On peut citer, parmi les pigments et les nanopigments minéraux, les dioxydes de titane, de zirconium ou de cérium, ainsi que les oxydes de zinc, de fer ou de chrome, les nanotitanes, le bleu ferrique. Parmi les pigments organiques, on peut citer le noir de carbone, et les laques couramment employées pour conférer aux lèvres et à la peau un effet de maquillage, qui sont des sels de calcium, de baryum, d'aluminium ou de zirconium, de colorants acides tels que les colorants halogéno-acides, azoïques ou anthraquinoniques.

Les pigments peuvent notamment être enrobés par des composés siliconés tels que des PDMS et/ou par des polymères, notamment des polyéthylènes ou encore par des composés fluorés. On peut ainsi citer les pigments SA de Maprecos ou les pigments PI de Myoshi.

Par nacres, il faut comprendre des particules irisées qui réfléchissent la lumière.

Les nacres peuvent être présentes dans le gel à raison de 0-40% en poids, de préférence à raison de 0,1 à 30% et de préférence encore à raison de 1-20% en poids.

Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.

Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage.

Les charges, qui peuvent être présentes dans le gel à raison de 0-60 % en poids, par rapport au poids total du gel, de préférence à raison de 0,1 à 40%, de préférence encore 1-20%, peuvent être minérales ou de synthèse, lamellaires ou non lamellaires.

On peut citer le talc, le mica, la silice, le kaolin, les poudres de Nylon, de poly-β-alanine et de polyéthylène, le Téflon, la lauroyl-lysine, l'amidon, le nitrure de bore, l'oxychlorure de bismuth, les poudres de polymères de tétrafluoroéthylène, les poudres de polyméthylméthacrylate, les poudres de polyuréthanne, les poudres de polystyrène, les poudres de polyester, les microsphères creuses synthétiques, les microéponges, les microbilles de résine de silicone, les oxydes de zinc et de titane, les oxydes de zirconium ou de cérium, le carbonate de calcium précipité, le carbonate et l'hydrocarbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone, comme le stéarate de zinc, de magnésium ou de lithium, le laurate de zinc, le myristate de magnésium, les composés SiO₂/TiO₂/ SiO₂, TiO₂/CeO₂/SiO₂, ou encore TiO₂/ZnO/Talc, les polymères de polyéthylène terephtalate/polyméthacrylate en forme de paillettes.

De manière générale, la phase pulvérulente comprend suffisamment de pigments et/ou nacres et/ou charges pour assurer l'effet maquillage souhaité. Ainsi, de préférence, le gel aqueux selon l'invention n'est pas transparent, c'est-à-dire qu'on ne peut pas voir les caractères d'une page de journal à travers le gel. De préférence encore il n'est pas translucide, c'est-à-dire qu'il ne permet pas le passage de la lumière.

Les gels de l'invention peuvent être incorporés au sein de compositions cosmétiques et en constituer la phase continue. De telles compositions peuvent ainsi comprendre une phase grasse qui peut par exemple comprendre une huile.

Parmi les huiles utilisables, on peut citer les huiles d'origine animale, végétale ou minérale, telles que l'huile de paraffine, de vaseline, le perhydrosqualène, l'huile d'abricot, l'huile de germes de blé, d'amande douce, de calophyllum, de sésame, de macadamia, de pépins de raisin, de colza, de coprah, d'arachide, de palme, de ricin, d'avocat, de jojoba, d'olive ou de germes de céréales; des esters d'acides gras et de polyol, en particulier les triglycérides liquides; des alcools; des acétylglycérides; des octanoates, décanoates ou ricinoléates d'alcools ou de polyalcools; des triglycérides d'acides gras; des glycérides, les huiles fluorées et perfluorées; les huiles de synthèse telles que les esters gras; les huiles de silicone telles que les huiles de silicone volatile, les polyméthylsiloxanes, les polyméthylphénylsiloxanes, les polysiloxanes modifiés par des acides gras, des alcools gras ou des polyoxyalkylènes, les silicones fluorées et les huiles perfluorées.

La phase grasse des compositions selon l'invention peut comprendre en outre d'autres corps gras, qui peuvent être choisis par l'homme du métier sur base de ses connaissances générales, de manière à conférer à la composition finale les propriétés souhaitées, par exemple en consistance, en texture et/ou en transfert. Ces corps gras additionnels peuvent être des cires, des gommes et/ou des corps gras pâteux d'origine animale, végétale, minérale ou synthétique, ainsi que leurs mélanges.

On peut notamment citer :
- les gommes de silicones,
- les cires d'origine animale, végétale, minérale ou synthétique telles que les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25°C, les ozokérites, les esters gras et les glycérides concrets à 25°C; les cires de polyéthylène et les cires obtenues par synthèse de Fischer-Tropsch; des huiles hydrogénées concrètes à 25°C; des lanolines; des esters gras concrets à 25°C; les cires de silicone; les cires fluorées; leurs mélanges.

La phase grasse peut être présente dans des proportions allant, par exemple, jusqu'à 30 %, de préférence de 0,1 à 20% et mieux de 0,5 à 10 % du poids total de la composition, ces proportions variant selon l'application choisie.

Les huiles ou les cires peuvent être introduites dans la phase aqueuse en présence d'un ou de plusieurs tensioactifs pour assurer une meilleure dispersion.

Les compositions selon l'invention peuvent donc également contenir un ou plusieurs tensioactifs H/E ou cotensio-actifs ioniques ou non ioniques , de HLB (hydrophile-lipophile balance) supérieur ou égal à 8, habituellement utilisés dans le domaine cosmétique. Lorsqu'il est présent, la quantité d'agent tensioactif ou de cotensio-actif va de préférence de 0,05 à 8 % du poids total de la composition.

La composition peut comprendre en outre tout composé complémentaire usuellement utilisé dans le domaine cosmétique. Ces composés complémentaires peuvent être choisis parmi les antioxydants, les huiles essentielles, les conservateurs, les actifs cosmétiques ou pharmaceutiques lipophiles ou hydrophiles, les hydratants, les vitamines, les acides gras essentiels, les sphingolipides, les composés auto-bronzants tels que la DHA, les filtres solaires, les parfums, et leurs mélanges.

Bien entendu l'homme du métier veillera à choisir ce ou ces éventuels composés complémentaires, et/ou leur quantité, de manière telle que les propriétés avantageuses du gel et/ou de la composition selon l'invention ne soient pas, ou substantiellement pas, altérées par l'adjonction envisagée.

Les gels et les compositions à phase continue aqueuse selon l'invention peuvent être préparés selon les méthodes de préparation classiques des sticks, ces méthodes étant bien connues de l'homme du métier.

Les gels et les compositions selon l'invention peuvent constituer des produits de maquillage ou de soin de la peau, en particulier du corps, du visage et/ou du cuir chevelu, ou des fibres kératiniques, en particulier des cheveux, des ongles, des cils et/ou des sourcils, ou encore des muqueuses, en particulier des lèvres. Ils peuvent ainsi constituer des produits de maquillage du corps, des fonds de teints, des ombres à paupières, des fards à joues, des anti-cemes, des rouges à lèvres, des crayons du contour des lèvres, des mascaras, des crayons du contour des yeux, des sticks pour la teinture ou le maquillage de mèches de cheveux.

L'invention est illustrée plus en détails dans les exemples suivants.

Dans les exemples suivants, les quantités sont données en pourcentage en poids par rapport au poids total de la composition.

### EXEMPLE 1 :

La Demanderesse a réalisé le gel aqueux sous forme de fond de teint en stick suivant :
- Gomme de gellane vendue sous la dénomination commerciale « Kelcogel F » de NUTRASWEET-KELCO 0,5%
- chlorure de Mg 0.1%
- PEG-12 5 %
- conservateur qs
- pigments (oxydes de fer et dioxyde de titane) 14.%
- propylène glycol 7.%
- eau qsp 100 %

Ce gel a été préparé de la manière suivante : l'eau et le conservateur ont été chauffés à 90 °C, puis on a incorporé le gellane sous agitation. Après avoir attendu 15 mn, on a incorporé le MgCl₂ et le PEG-12 sous agitation, puis la pâte pigmentaire réalisée préalablement en mélangeant les pigments au propylène glycol.

Le mélange a ensuite été coulé en coupelle puis refroidi. Le tout est laissé au repos 24 h à température ambiante.

On obtient un stick très frais à l'application, de bonne solidité, de bonne prise et d'application facile et homogène sur la peau.

Le maquillage de la peau avec un tel stick donne un résultat naturel et transparent, totalement dépourvu d'effet gras.

La dureté de ce gel, mesurée comme décrit ci-dessus est de : 19 g.

Ce stick se délite très bien.

### EXEMPLE 2 : comparatif

La Demanderesse a réalisé le même stick que dans l'exemple 1 mais en remplaçant les 5% de PEG-12 par 5% de PEG-8.

### Le stick obtenu présente une dureté mesurée comme décrit ci-dessus de : 13,1 g

Ce stick est de texture trop caoutchouteuse pour permettre un délitage satisfaisant.

### EXEMPLE 3 :

La Demanderesse a réalisé le même stick que dans l'exemple 1 mais en remplaçant les 5% de PEG-12 par 5% de PEG-180.

Le stick obtenu présente une dureté mesurée comme décrit ci-dessus de 20,7 g;

Il est facile à déliter. Il permet un maquillage homogène et uniforme directement sur la peau, au doigt ou à l'éponge.

### EXEMPLE 4 : comparatif

La Demanderesse a réalisé le même stick que dans l'exemple 1 mais en remplaçant les 5% de PEG-12 par 5% de PEG-115M dont le nombre de moles d'oxyéthylène est de 115 000.

Le stick obtenu est extrêmement mou : sa dureté ne peut pas être mesurée. Ce produit est très collant et est difficilement applicable sur la peau.

## Revendications

1. Gel aqueux solide comprenant i) au moins un gélifiant hydrophile et ii) au moins un polyéthylène glycol dont le nombre de moles d'oxyéthylène va de 12 à 180.

2. Gel selon la revendication 1, **caractérisé en ce que** le gélifiant hydrophile est choisi parmi les polysaccharides, les dérivés de protéines, les gels de synthèse ou d'hémisynthèse de type polyester, en particulier sulfonique, les polyacrylates ou polyméthacrylates et leurs dérivés.

3. Gel selon la revendication 2, **caractérisé en ce que** le gélifiant hydrophile est un polysaccharide choisi parmi:
- les extraits d'algue tels que l'agar-agar, les carraghénanes, les alginates, en particulier de Na ou Ca;
- les exsudats de micro-organismes tels que la gomme de xanthane et ses dérivés ou encore la gomme de gellane,
- les extraits de fruits tels que les pectines ;
- les agents gélifiants d'origine animale comme les dérivés de protéine, en particulier la gélatine, de boeuf ou de poisson, les caséïnates ;
- les polysaccharides possédant une chaîne latérale et 6 sucres neutres,
- et leurs mélanges.

4. Gel selon la revendication 3, **caractérisé en ce que** le gélifiant hydrophile est choisi parmi le gellane, les caraghénanes et leurs mélanges.

5. Gel selon l'une quelconque des revendications précédentes, **caractérisé en ce que** le gélifiant hydrophile est présent à une concentration allant de 0,1 à 30%, de préférence de 0,2 à 10%, en poids, par rapport au poids total du gel.

6. Gel selon l'une quelconque des revendications précédentes, **caractérisé en ce que** le polyéthylène glycol présente un nombre de moles d'oxyéthylène de 12.

7. Gel selon l'une quelconque des revendications précédentes, **caractérisé en ce que** le polyéthylène glycol est présent à une concentration allant de 1 à 20% en poids, de préférence encore de 2 à 10% en poids, par rapport au poids total du gel.

8. Gel selon l'une quelconque des revendications précédentes, **caractérisé en ce qu'**il comprend en outre une phase pulvérulente comprenant un pigment et/ou une nacre et/ou une charge.

9. Gel selon la revendication 8, **caractérisé en ce que** les pigments sont choisis parmi les dioxydes de titane, de zirconium ou de cérium, les oxydes de zinc, de fer ou de chrome, les nanotitanes, le bleu ferrique, le noir de carbone, les sels de calcium, de baryum, d'aluminium ou de zirconium, de colorants acides tels que les colorants halogéno-acides, azoïques ou anthraquinoniques, les pigments enrobés par des composés siliconés tels que des polydiméthylsiloxanes et/ou par des polymères, notamment des polyéthylènes, ou encore par des composés fluorés, et/ou leurs mélanges.

10. Gel selon l'une quelconque des revendications 8 ou 9, **caractérisé en ce que** les pigments sont présents à une teneur allant jusqu'à 40%, en poids, de préférence de 0,1 à 30%, en poids, par rapport au poids total du gel.

11. Gel selon l'une quelconque des revendications 8 à 10, **caractérisé en ce que** les nacres sont choisies parmi la nacre naturelle, le mica recouvert d'oxyde de titane, d'oxyde de fer, de pigment naturel ou d'oxychlorure de bismuth ainsi que le mica titane coloré.

12. Gel selon l'une quelconque des revendications 8 à 11, **caractérisé en ce que** les nacres sont présentes à une teneur allant jusqu'à 40%, en poids, de préférence de 0,1 à 30%, en poids, par rapport au poids total du gel.

13. Gel selon l'une quelconque des revendications 8 à 12, **caractérisé en ce que** les charges sont choisies parmi le talc, le mica, la silice, le kaolin, les poudres de Nylon, de poly-β-alanine et de polyéthylène, le Téflon, la lauroyl-lysine, l'amidon, le nitrure de bore, l'oxychlorure de bismuth, les poudres de polymères de tétrafluoroéthylène, les poudres de polyméthylméthacrylate, les poudres de polyuréthanne, les poudres de polystyrène, les poudres de polyester, les microsphères creuses synthétiques, les microéponges, les microbilles de résine de silicone, les oxydes de zinc et de titane, les oxydes de zirconium ou de cérium, le carbonate de calcium précipité, le carbonate et l'hydrocarbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone, comme le stéarate de zinc, de magnésium ou de lithium, le laurate de zinc, le myristate de magnésium, les composés SiO₂/TiO₂/ SiO₂, TiO₂/CeO₂/SiO₂, ou encore TiO₂/ZnO/Talc, les polymères de polyéthylène terephtalate/polyméthacrylate en forme de paillettes.

14. Gel selon l'une quelconque des revendications 8 à 13, **caractérisé en ce que** les charges sont présentes à une teneur allant jusqu'à 60% en poids, de préférence de 0,1 à 40%, en poids, par rapport au poids total du gel.

15. Gel selon l'une quelconque des revendications précédentes, **caractérisé en ce qu'**il comprend en outre un sel.

16. Gel selon la revendication précédente, **caractérisé en ce que** le sel est choisi parmi le nitrate de calcium, de magnésium ou de strontium, le borate de calcium ou de magnésium, le chlorure de calcium, de sodium, de magnésium, de strontium, de néodyrne ou de manganèse, le sulfate de magnésium ou de calcium, l'acétate de calcium ou de magnésium, et leurs mélanges.

17. Gel selon la revendication précédente, **caractérisé en ce que** le sel est le chlorure de magnésium.

18. Gel selon l'une quelconque des revendications précédentes, **caractérisé en ce qu'**il comprend en outre un milieu cosmétiquement ou physiologiquement acceptable.

19. Gel selon l'une quelconque des revendications précédentes, **caractérisé en ce qu'**il comprend en outre un colorant hydrosoluble.

20. Gel selon l'une quelconque des revendications précédentes, **caractérisé en ce qu'**il comprend en outre un solvant choisi parmi l'éthanol, l'isopropanol, le propylène glycol, le butylène glycol, le dipropylène glycol, le diéthylène glycol, les éthers de glycol, et leurs mélanges.

21. Gel selon l'une quelconque des revendications précédentes, **caractérisé en ce qu'**il comprend en outre un composé complémentaire choisi parmi les antioxydants, les huiles essentielles, les conservateurs, les actifs cosmétiques ou pharmaceutiques lipophiles ou hydrophiles, les hydratants, les vitamines, les acides gras essentiels, les sphingolipides, les composés auto-bronzants, les filtres solaires, les parfums, et leurs mélanges.

22. Composition solide à phase continue aqueuse, **caractérisée en ce qu'**elle comprend un gel tel que défini à l'une quelconque des revendications 1 à 21.

23. Produit de maquillage de la peau ou des fibres kératiniques, **caractérisé en ce qu'**il comprend un gel tel que défini à l'une quelconque des revendications 1 à 21 et/ou une composition telle que définie à la revendication 22.

24. Produit selon la revendication 23, **caractérisé en ce qu'**il constitue un produit de maquillage du corps, un fond de teint, une ombre à paupières, un fards à joues, un anti-cernes, un rouge à lèvres, un crayon du contour des lèvres, un mascara, un crayon du contour des yeux, un stick pour la teinture ou le maquillage de mèches de cheveux.

25. Procédé de maquillage de la peau et/ou des fibres kératiniques, consistant à appliquer sur la peau et/ou les fibres kératiniques, un gel tel que défini à l'une quelconque des revendications 1 à 21 et/ou une composition telle que définie à la revendication 22 et/ou un produit tel que défini à l'une quelconque des revendications 23 ou 24.

## Claims

1. Solid aqueous gel comprising i) at least one hydrophilic gelling agent and ii) at least one polyethylene glycol in which the number of moles of oxyethylene ranges from 12 to 180.

2. Gel according to Claim 1, **characterized in that** the hydrophilic gelling agent is selected from polysaccharides, protein derivatives, synthetic or semisynthetic gels of polyester type, especially sulphonic type, polyacrylates or polymethacrylates and derivatives thereof.

3. Gel according to Claim 2, **characterized in that** the hydrophilic gelling agent is a polysaccharide selected from:
- algal extracts such as agar, carragheenans, alginates, especially those of Na or Ca;
- exudates of microorganisms, such as xanthan gum and its derivatives or else gellan gum,
- fruit extracts such as pectins;
- gelling agents of animal origin such as protein derivatives, especially gelatin, from cattle or fish, and caseinates;
- polysaccharides possessing a side chain and 6 neutral sugars,
- and mixtures thereof.

4. Gel according to Claim 3, **characterized in that** the hydrophilic gelling agent is selected from gellan, carragheenans and mixtures thereof.

5. Gel according to any one of the preceding claims, **characterized in that** the hydrophilic gelling agent is present at a concentration ranging from 0.1 to 30%, preferably from 0.2 to 10%, by weight, relative to the total weight of the gel.

6. Gel according to any one of the preceding claims, **characterized in that** the polyethylene glycol has a number of moles of oxyethylene of 12.

7. Gel according to any one of the preceding claims, **characterized in that** the polyethylene glycol is present at a concentration ranging from 1 to 20% by weight, more preferably from 2 to 10% by weight, relative to the total weight of the gel.

8. Gel according to any one of the preceding claims, **characterized in that** it additionally comprises a pulverulent phase comprising a pigment and/or a nacreous substance and/or a filler.

9. Gel according to Claim 8, **characterized in that** the pigments are selected from titanium, zirconium or cerium dioxides, zinc, iron or chromium oxides, nanotitaniums, ferric blue, carbon black, calcium, barium, aluminium or zirconium salts, acid dyes such as halo-acid dyes, azo dyes or anthraquinonoid dyes, pigments coated with silicone compounds such as polydimethylsiloxanes and/or with polymers, especially polyethylenes, or else with fluorinated compounds, and/or mixtures thereof.

10. Gel according to either of Claims 8 and 9, **characterized in that** the pigments are present in an amount ranging up to 40% by weight, preferably from 0.1 to 30% by weight, relative to the total weight of the gel.

11. Gel according to any one. of Claims 8 to 10, **characterized in that** the nacreous substances are selected from natural nacre, mica covered with titanium oxide, iron oxide, natural pigment or bismuth oxychloride, and coloured titanium mica.

12. Gel according to any one of Claims 8 to 11, **characterized in that** the nacreous substances are present in an amount ranging up to 40% by weight, preferably from 0.1 to 30% by weight, relative to the total weight of the gel.

13. Gel according to any one of Claims 8 to 12, **characterized in that**. the fillers are selected from talc, mica, silica, kaolin, powders of Nylon, poly-β-alanine and polyethylene, Teflon, lauroyllysine, starch, boron nitride, bismuth oxychloride, tetrafluoroethylene polymer powders, polymethyl methacrylate powders, polyurethane powders, polystyrene powders, polyester powders, synthetic hollow microspheres, microsponges, silicone resin microbeads, oxides of zinc and of titanium, oxides of zirconium or of cerium, precipitated calcium carbonate, magnesium carbonate and basic magnesium carbonate, hydroxyapatite, hollow silica microspheres, glass or ceramic microcapsules, metal soaps derived from organic carboxylic acids having 8 to 22 carbon atoms, preferably 12 to 18 carbon atoms, 'such as zinc stearate, magnesium stearate or lithium stearate, zinc laurate and magnesium myristate, the compounds SiO₂/TiO₂/SiO₂, TiO₂/CeO₂/SiO₂, or else TiO₂/ZnO/talc, and polyethylene terephthalate/polymethacrylate polymers in the form of flakes.

14. Gel according to any one of Claims 8 to 13, **characterized in that** the fillers are present in an amount ranging up to 60% by weight, preferably from 0.1 to 40% by weight, relative to the total weight of the gel.

15. Gel according to any one of the preceding claims, **characterized in that** it further comprises a salt.

16. Gel according to the preceding claim, **characterized in that** the salt is selected from calcium, magnesium or strontium nitrate, calcium or magnesium borate, calcium, sodium, magnesium, strontium, neodymium or manganese chloride, magnesium or calcium sulphate, calcium or magnesium acetate, and mixtures thereof.

17. Gel according to the preceding claim, **characterized in that** the salt is magnesium chloride.

18. Gel according to any one of the preceding claims, **characterized in that** it further comprises a cosmetically or physiologically acceptable medium.

19. Gel according to any one of the preceding claims, **characterized in that** it further comprises a water-soluble dye.

20. Gel according to any one of the preceding claims, **characterized in that** it further comprises a solvent selected from ethanol, isopropanol, propylene glycol, butylene glycol, dipropylene glycol, diethylene glycol, glycol ethers, and mixtures thereof.

21. Gel according to any one of the preceding claims, **characterized in that** it further comprises an additional compound selected from antioxidants, essential oils, preservatives, active lipophilic or hydrophilic pharmaceutical or cosmetic substances, moisturizers, vitamins, essential fatty acids, sphingolipids, self-tanning compounds, sunscreens, fragrances, and mixtures thereof.

22. Solid composition with a continuous aqueous phase, **characterized in that** it comprises a gel as defined in any one of Claims 1 to 21.

23. Makeup product for the skin or keratinous fibres, **characterized in that** it comprises a gel as defined in any one of Claims 1 to 21 and/or a composition as defined in Claim 22.

24. Product according to Claim 23, **characterized in that** it constitutes a body makeup product, a foundation, an eyeshadow, a blusher, a concealer, a lipstick, a lipliner pencil, a mascara, an eyeliner pencil, or a stick for colouring or making up locks of hair.

25. Method of making up the skin and/or keratinous fibres, which consists in applying to the skin and/or keratinous fibres a gel as defined in any one of Claims 1 to 21 and/or a composition as defined in Claim 22 and/or a product as defined in either of Claims 23 and 24.

## Patentansprüche

1. Gel, das wässerig und fest ist und das i) mindestens einen hydrophilen Gelbildner und ii) mindestens ein Polyethylenglykol mit 12 bis 180 mol Ethylenoxid enthält.

2. Gel nach Anspruch 1, **dadurch gekennzeichnet, dass** der hydrophile Gelbildner unter Polysacchariden, Proteinderivaten, synthetischen oder halbsynthetischen Gelen vom Typ eines Polyesters und insbesondere eines Sulfopolyesters, Polyacrylaten oder Polymethacrylaten und den Derivaten dieser Verbindungen ausgewählt ist.

3. Gel nach Anspruch 2, **dadurch gekennzeichnet, dass** der hydrophile Gelbildner ein Polysaccharid ist, das ausgewählt ist unter:
- Algenextrakten, wie Agar-Agar, Carrageenanen, Alginaten und insbesondere Natriumalginat oder Calciumalginat,
- Exsudaten von Mikroorganismen, wie Xanthangummi und seinen Derivaten oder auch Gellangummi,
- Fruchtextrakten, wie Pektinen,
- Gelbildnern tierischen Ursprungs, wie Proteinderivaten und insbesondere Gelatine von Rindern oder Fischen sowie Caseinaten,
- Polysacchariden, die eine Seitenkette und 6 neutrale Zucker aufweisen, sowie
- den Gemischen dieser Verbindungen.

4. Gel nach Anspruch 3, **dadurch gekennzeichnet, dass** der hydrophile Gelbildner unter Gellan, Carrageenanen und deren Gemischen ausgewählt ist.

5. Gel nach einem der vorhergehenden Ansprüche, **dadurch gekennzeichnet, dass** der hydrophile Gelbildner in einer Konzentration von 0,1 bis 30 Gew.-% und vorzugsweise von 0,2 bis 10 Gew.-%, bezogen auf das Gesamtgewicht des Gels, vorliegt.

6. Gel nach einem der vorhergehenden Ansprüche, **dadurch gekennzeichnet, dass** das Polyethylenglykol 12 mol Ethylenoxid aufweist.

7. Gel nach einem der vorhergehenden Ansprüche, **dadurch gekennzeichnet, dass** das Polyethylenglykol in einer Konzentration von 1 bis 20 Gew.-% und vorzugsweise von 2 bis 10 Gew.-%, bezogen auf das Gesamtgewicht des Gels, vorliegt.

8. Gel nach einem der vorhergehenden Ansprüche, **dadurch gekennzeichnet, dass** es ferner eine pulverförmige Phase aufweist, die ein Pigment und/oder ein Perlglanzmittel und/oder einen Füllstoff enthält.

9. Gel nach Anspruch 8, **dadurch gekennzeichnet, dass** die Pigmente unter Titandioxid, Zirconiumdioxid, Cerdioxid, Zinkoxid, Eisenoxid, Chromoxid, Nanotitanpigmenten, Eisenblau, Ruß, Calciumsalzen, Bariumsalzen, Aluminiumsalzen, Zirconiumsalzen, sauren Farbstoffen, wie Halogen-Säurefarbstoffen, Azofarbstoffen oder Anthrachinonfarbstoffen, mit Siliconverbindungen, wie Polydimethylsiloxanen, und/oder mit Polymeren, insbesondere Polyethylenen, oder auch mit fluorierten Verbindungen umhüllten Pigmenten und/oder den Gemischen dieser Verbindungen ausgewählt sind.

10. Gel nach einem der Ansprüche 8 oder 9, **dadurch gekennzeichnet, dass** die Pigmente in einem Mengenanteil von bis zu 40 Gew.-% und vorzugsweise von 0,1 bis 30 Gew.-%, bezogen auf das Gesamtgewicht des Gels, vorliegen.

11. Gel nach einem der Ansprüche 8 bis 10, **dadurch gekennzeichnet, dass** die Perlglanzmittel unter natürlicher Perlmutter, mit Titandioxid, Eisenoxid, natürlichem Pigment oder Bismutoxidchlorid überzogenem Glimmer sowie farbigem Titanglimmer ausgewählt sind.

12. Gel nach einem der Ansprüche 8 bis 11, **dadurch gekennzeichnet, dass** die Perlglanzmittel in einem Mengenanteil von bis zu 40 Gew.-% und vorzugsweise von 0,1 bis 30 Gew.-%, bezogen auf das Gesamtgewicht des Gels, vorliegen.

13. Gel nach einem der Ansprüche 8 bis 12, **dadurch gekennzeichnet, dass** die Füllstoffe unter Talk, Glimmer, Kieselsäuren, Kaolin, Nylonpulvern, Poly(β-alanin)pulvern, Polyethylenpulvern, Teflon, Lauroyllysin, Stärke, Bornitrid, Bismutoxidchlorid, Polytetrafluorethylenpulvern, Polymethylmethacrylatpulvern, Polyurethanpulvern, Polystyrolpulvern, Polyesterpulvern, synthetischen Mikrohohlkugeln, Mikroschwämmen, Mikrokugeln aus Siliconharz, den Oxiden von Zink und Titan, den Oxiden von Zirconium oder Cer, gefälltem Calciumcarbonat, Magnesiumcarbonat, Magnesiumhydrocarbonat, Hydroxylapatit, Mikrohohlkugeln aus Kieselsäuren, Mikrokapseln aus Glas oder aus Keramik, Metallseifen, die von organischen Carbonsäuren mit 8 bis 22 Kohlenstoffatomen und vorzugsweise 12 bis 18 Kohlenstoffatomen abgeleitet sind, wie Zinkstearat, Magnesiumstearat, Lithiumstearat, Zinklaurat und Magnesiummyristat, den Verbindungen SiO₂/TiO₂/SiO₂, TiO₂/CeO₂/SiO₂ oder auch TiO₂/ ZnO/Talk sowie den Polyethylenterephthalat/Polymethacrylat-Polymeren in Form von Plättchen ausgewählt sind.

14. Gel nach einem der Ansprüche 8 bis 13, **dadurch gekennzeichnet, dass** die Füllstoffe in einem Mengenanteil von bis zu 60 Gew.-% und vorzugsweise von 0,1 bis 40 Gew.-%, bezogen auf das Gesamtgewicht des Gels, vorliegen.

15. Gel nach einem der vorhergehenden Ansprüche, **dadurch gekennzeichnet, dass** es ferner ein Salz enthält.

16. Gel nach dem vorhergehenden Anspruch, **dadurch gekennzeichnet, dass** das Salz unter Calciumnitrat, Magnesiumnitrat, Strontiumnitrat, Calciumborat, Magnesiumborat, Calciumchlorid, Natriumchlorid, Magnesiumchlorid, Strontiumchlorid, Neodymchlorid, Manganchlorid, Magnesiumsulfat, Calciumsulfat, Calciumacetat, Magnesiumacetat und den Gemischen dieser Verbindungen ausgewählt ist.

17. Gel nach dem vorhergehenden Anspruch, **dadurch gekennzeichnet, dass** es sich bei dem Salz um Magnesiumchlorid handelt.

18. Gel nach einem der vorhergehenden Ansprüche, **dadurch gekennzeichnet, dass** es ferner ein kosmetisch oder physiologisch akzeptables Medium enthält.

19. Gel nach einem der vorhergehenden Ansprüche, **dadurch gekennzeichnet, dass** es ferner einen wasserlöslichen Farbstoff enthält.

20. Gel nach einem der vorhergehenden Ansprüche, **dadurch gekennzeichnet, dass** es ferner ein Lösemittel enthält, das unter Ethanol, Isopropanol, Propylenglykol, Butylenglykol, Dipropylenglykol, Diethylenglykol, Glykolethern und den Gemischen dieser Verbindungen ausgewählt ist.

21. Gel nach einem der vorhergehenden Ansprüche, **dadurch gekennzeichnet, dass** es ferner eine ergänzende Verbindung enthält, die unter Antioxidantien, etherischen Ölen, Konservierungsstoffen, lipophilen oder hydrophilen kosmetischen oder pharmazeutischen Wirkstoffen, Hydratisierungsmitteln, Vitaminen, essentiellen Fettsäuren, Sphingolipiden, selbstbräunenden Verbindungen, Sonnenschutzfiltern, Parfums und den Gemischen dieser Verbindungen ausgewählt ist.

22. Feste Zusammensetzung mit kontinuierlicher wässeriger Phase, **dadurch gekennzeichnet, dass** sie ein Gel nach einem der Ansprüche 1 bis 21 enthält.

23. Produkt zum Schminken der Haut oder keratinischer Fasern, **dadurch gekennzeichnet, dass** es ein Gel nach einem der Ansprüche 1 bis 21 und/oder eine Zusammensetzung nach Anspruch 22 enthält.

24. Produkt nach Anspruch 23, **dadurch gekennzeichnet, dass** es sich um ein Produkt zum Schminken des Körpers, ein Make up, einen Lidschatten, ein Wangenrouge, ein Produkt zum Abdecken von Augenringen, einen Lippenstift, einen Lippenkonturstift, eine Mascara, einen Augenkonturstift oder um einen Stick zum Färben oder zum Schminken von Haarsträhnen handelt.

25. Verfahren zum Schminken der Haut und/oder keratinischer Fasern, das darin besteht, ein Gel nach einem der Ansprüche 1 bis 21 und/oder eine Zusammensetzung nach Anspruch 22 und/oder ein Produkt nach einem der Ansprüche 23 oder 24 auf die Haut und/oder die keratinischen Fasern aufzubringen.
